# EUROPÄISCHE PATENTSCHRIFT

(11) **EP 1 355 616 B1**
(45) Veröffentlichungstag und Bekanntmachung des Hinweises auf die Patenterteilung: **14.02.2007**
(21) Anmeldenummer: 02712729.9
(22) Anmeldetag: 29.01.2002
(51) Int. Cl.: A61Q 19/00, A61K 8/14, A61K 8/44, A61K 8/49, A61K 8/55, A61K 8/60, A61K 8/73

(54) **REVITALISIERENDER WIRKKOMPLEX FÜR DIE HAUT**
REVITALISING ACTIVE COMPLEX FOR THE SKIN
COMPLEXE ACTIF REVITALISANT POUR LA PEAU

(30) Priorität: 02.02.2001 DE 10106288
(43) Veröffentlichungstag der Anmeldung: 29.10.2003
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: PCT/DE2002/000363
(87) Internationale Veröffentlichungsnummer: WO 2002/060394

(56) Entgegenhaltungen:
- WO-A-99/21531
- DE-A- 19 841 385
- US-A- 4 582 807

## Beschreibung

Die Erfindung betrifft einen revitalisierenden Wirkkomplex für die Haut und damit hergestellte kosmetische Erzeugnisse.

Aus der DE-A-19841385 ist die Verwendung von Kreatin oder Kreatinderivaten als Feuchthaltemittel in kosmetischen Zubereitungen bekannt. Dabei können entsprechende Produkte sowohl oral eingenommen als auch auf die Haut lokal aufgetragen werden. Durch die lokale Anwendung soll die Hautaustrocknung zurückgedrängt werden.

Es ist weiterhin bekannt, bestimmte Glucane kosmetisch einzusetzen. β-(1,3)-Glucane werden gemäß DE-A-19911058 in feinteiliger Form zur besseren Resorption im Stratum corneum der Haut verwendet. Aus Getreide hergestelltes β-D-Glucan wird gemäß WO99/21531 für die Behandlung von Verbrennungen und Wundnarben eingesetzt. Wasserunlösliche lineare Polyglucane werden gemäß DE -A-19860371 für den Erhalt eines besonders angenehmen Hautgefühls vorgeschlagen.

Der Erfindung liegt die Aufgabe zugrunde, neue kosmetische Mittel mit insbesondere deutlich verbesserter revitalisierender Hautwirkung bereitzustellen.

Erfindungsgemäß bereitgestellt wird ein revitalisierender Wirkkomplex für die Haut, der dadurch gekennzeichnet ist, daß der Komplex besteht aus
- 0,5 bis 9 Gew-% Kreatin oder einem Kreatinderivat
- 0,1 bis 40 Gew-% wasserlöslichem Glykogen
- 0,1 bis 10 Gew-% eines Phospholipids
- 0,1 bis 5 Gew-% eines kosmetisch annehmbaren Gels, der Rest zu 100 Gew-% Wasser,
in homogener Verteilung des Gemisches Kreatin, Glykogen und Phospholipid in dem wäßrigen Gel.

Kreatin (oder N-Carbamidoyl-N-methylglycin), das in wäßriger Lösung in Kreatinin (2-Imino-1-methylimidazolidin-4-on) übergeht, findet sich üblicherweise im Muskelsaft von Wirbeltieren und ist z.B. in Fleischextrakten enthalten. Derivate von Kreatin sind z.B. Kreatinphosphat, Kreatincitrat oder Kreatinpyruvat oder Gemische davon. Das Ascorbat oder das Monohydrat können ebenfalls eingesetzt werden, sind jedoch nicht bevorzugt.

Glykogen gehört zu den verzweigten Glucanen und ist ein α-D-1,4-Glucan mit Verzweigungen über α-1,6-Bindungen. Es kann aus Muskelgewebe oder auch aus Hefen gewonnen werden. Ein bevorzugtes Glykogen für den erfindungsgemäßen Einsatz ist ein solches mit einer Dichte bei 20 °C von etwa 1,14 und einem Brechungsindex bei 20 °C von 1,399 bis 1,404 und einem Trokkenextrakt (Einwaage 5 g) von 39,0-48,0 %, insbesondere das Produkt Dermosaccharides GY^{®} aus maritimem Ursprung. Das Glykogen hat vorzugsweise ein Molgewicht von 2,7•10⁶ bis 1•10⁸ Dalton.

Bevorzugte Anteile Glykogen liegen im Bereich von 0,5 bis 15 Gew-%.

Als Phospholipide können z.B. eingesetzt werden Phosphatidylcholin, Phosphatidylethanolamin, Phosphatidylinositol, Phosphatidylserin, Phosphatidsäure und Lysolecithine sowie Gemische davon. Bekannte Produkte sind beispielsweise Phospholipon^{®}80/80H, Phospholipon^{®}9oG/H.

Bevorzugte Anteile Phospholide liegen im Bereich von 0,5 bis 8 Gew-%.

Zu geeigneten Gelbildnern für das im Wirkkomplex enthaltene Gel gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon, Montmorillonit. Carbomer und Guargummi sind bevorzugt.

Der erfindungsgemäße Wirkkomplex reduziert die LDH-Freisetzung (LDH = Lacto-deshydrogenase, ein Stress-Enzym, das von den Zellen bei UV-B-Bestrahlung freigesetzt wird). Weiterhin erhöht sich bei Behandlung der Zellen mit dem Komplex der Sauerstoffverbrauch als Zeichen der Zellvitalität, und es erfolgt eine Zellreparatur, wodurch die Zellen jünger erscheinen und in besserer Erscheinungsform.

Die auftretende Wirkung in diesem Sinne ist überraschenderweise eine synergistische Wirkung, da die summierten Einzelwirkungen von Glykogen und Kreatin sowohl beim Anti-UV-Test als auch beim Sauerstoffverbrauchstest deutlich übertroffen werden. Es war weiterhin überraschend, daß der Synergismus noch ein weiteres mal erhöht werden kann durch den Zusatz von 0,05 bis 10 Gew-% eines β-1,3-Glucans, das durch ein Ultraschall-Aufschlußverfahren von Hefe, beispielsweise Bäckerhefe oder Bierhefe, gemäß US-A-5,629,185 gewonnen wurde. Bei diesem Verfahren wird in einer Ultraschall-Durchflußzelle eine Hefesuspension durch einen Beschallungsraum geführt, bei dem die Sonotrode zur Hälfte bis Zweidrittel ihrer Länge in die Durchflußzelle hineinragt und in das zu beschallende Medium eintaucht. Dabei hat die Mittelachse der Sonotrode einen Winkel von 80,5 bis 88,5° zur Eintrittsebene in die Zelle, und das Verhältnis der Eintauchlänge der Sonotrode in mm zum Beschallungsvolumen in ml wird auf einen Wert von 1:1,1 bis 1:20 eingestellt. Der Feststoffanteil in dem zu beschallenden Medium liegt im Bereich von 1:0,02 bis 1:2,2 (in Gew.-%).

Der erfindungsgemäße Wirkkomplex kann in kosmetischen Zubereitungen in einer Menge von 0,01 bis 22 Gew-% enthalten sein, vorzugsweise 0,5 bis 12 Gew-%, insbesondere 1 bis 8 Gew-%. Zu derartigen Zubereitungen gehören z.B. Sonnencremes, Sonnengele, After-sun-Produkte, Tagescremes, Nachtcremes, Masken, Körperlotionen, Reinigungsmilch, Make up's, Lippenstiften, Augenkosmetik, Haarmasken, Haarspülungen, Haarshampoos, Duschgelen, Duschölen, Badeölen. Die Herstellung derartiger Produkte erfolgt auf eine Weise, wie sie dem Fachmann auf diesem Gebiet bekannt ist.

Die Zubereitungen enthalten üblicherweise weitere Träger- und/oder Hilfsstoffe sowie gegebenenfalls weitere Wirkstoffe z.B. Wasser, Konservierungsmittel, Vitamine, Farbstoffe, Pigmente mit färbender Wirkung, Radikalfänger, Verdickungsmittel, weichmachende Substanzen, feuchthaltende Substanzen, Duftstoffe, Alkohole, Polyole, Ester, Elektrolyte, polare und unpolare öle, Polymere, Copolymere, Emulgatoren, Wachse, stabilisatoren.

Zu den kosmetischen Wirkstoffen gehören z. B. Emulgatoren, anorganische und organische Lichtschutzmittel, Radikalfänger, Feuchthaltemittel, Vitamine, Enzyme, pflanzliche Wirkstoffe, Polymere, Melanin, Antioxidationsmittel, entzündungswidrige natürliche Wirkstoffe, mit Sauerstoff beladene Fluorcarbone, mit Sauerstoff beladene asymmetrische lamellare Aggregate gemäß WO 94/00109, Kaolin sowie mit SiO₂ modifiziertes Kaolin gemäß WO94/17588, Anteile eines im wäßrigen Medium mild abgebauten und mit einer unterstöchiometrischen Menge eines C₁₀-C₂₀-Fettsäurehalogenids kondensierten Produktes aus pflanzlichem oder tierischem Ausgangsmaterial gemäß WO96/29048.

Weiterhin können Erweichungsmittel eingesetzt werden, wie stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, oleylalkohol, Isopropyllaurat, Decyloleat, octadecan-2-ol, Isocetylalkohol, Cetylpalmitat, Siliconöle wie Dimethylpolysiloxan, Isopropylmyristat, Isopropylpalmitat, Polyethylenglycol, Lanolin, Kakaobutter, pflanzliche öle wie Maisöl, Baumwollsamenöl, Olivenöl, Butylmyristat, Palmitinsäure.

Vorteilhaft für die kosmetische Zubereitungen mit dem erfindungsgemäßen Wirkkomplex kann es sein, daß die Zubereitung zusätzlich nach sauerstoffbeladene Fluorcarbone oder asymmetrische lamellare Aggregate enthält, wobei diese Aggregate aus Phospholipiden und mit Sauerstoff beladenen Fluorcarbonen oder Fluorcarbongemischen bestehen. Derartige asymmetrische lamellare Aggregate sind zum Beispiel aus der DE-B-42 21 255 oder der US-A-5643601 bekannt. Ihr Gehalt an Fluorcarbonen wie Perfluordecalin liegt im Bereich von 0,2 bis 100 % Gewicht/Volumen, wobei das Phospholipid einen Phosphatidylcholingehalt von mehr als 30 bis 99 Gew-% hat, und wobei diese Aggregate eine Hautpenetrierung in Abhängigkeit von der kritischen Löslichkeitstemperatur der Fluorcarbone besitzen.

Diese Aggregate sind Sauerstoffträger und ermöglichen ein Penetrieren des Sauerstoffs in die Haut und damit eine bessere Versorgung der Haut mit Sauerstoff. Die Herstellung dieser Aggregate erfolgt durch Hochdruckhomogenisierung von Phospholipiden, wie Sojalecithin und Eilecithin oder teilhydrierten Phospholipiden, die einen Phosphatidylcholingehalt von mehr als 30 Gew.-% bis 99 Gew.-% haben, mit perfluorierten oder hochfluorierten Kohlenstoffverbindungen oder Gemischen davon, die in der Lage sind, Gase, wie Sauerstoff und Kohlendioxid zu transportieren. Darin können neben Phosphatidylcholin auch Lysolecithine im Konzentrationsbereich von 0,1 bis 10 Gew.-% und/oder geladene Phospholipide wie Phosphatidylethanolamin, n-Acetylphosphatidylethanolamin oder Phosphatidsäure im Konzentrationsbereich 0,1 bis 30 Gew.-% vorhanden sein, bezogen auf das Gesamtgewicht der Aggregate.

Der Anteil der Aggregate kann im Bereich von 0,5 bis 40 Gew.-% liegen, bezogen auf die kosmetische Zubereitung, und liegt vorteilhaft im Bereich von 2 bis 15 Gew.-%, insbesondere im Bereich von 2 bis 10 Gew.-%.

Einen weiteren Wirkstoff, den eine kosmetische Zubereitung zusammen mit dem erfindungsgemäßen Wirkkomplex enthalten kann, sind fein verteilte hartmagnetische Einbereichsteilchen (Einkristalle) mit hoher Roerzitivfeldstärke von 3000 bis 5000 Oerstedt und mit Korngrößen im Bereich von 50 bis 1200 nm mit oder ohne die o.g. asymmetrischen lamellaren Aggregate, wobei diese hartmagnetischen Teilchen insbesondere Barium- und/oder Strontiumhexaferrite sind, erzeugt nach der Glaskristallisationstechnik durch Züchtung von Einkristallen aus einer abgeschreckten Glasschmelze (siehe US-A-5800835; US-A-5919490).

Der Anteil der hartmagnetischen Teilchen mit bevorzugten Teilchengrößen von 50-250 nm kann im Bereich von 0,5 bis 10 Gew.-% liegen, bezogen auf die kosmetische Zubereitung, und liegt vorteilhaft im Bereich von 1 bis 8 Gew.-%, insbesondere im Bereich von 1 bis 5 Gew.-%.

Besonders bevorzugt ist eine Ausführungsform der Erfindung, bei der der erfindungsgemäße Wirkkomplex zusammen mit sauerstoffbeladenen asymmetrischen lamellaren Aggregaten und hartmagnetischen Teilchen in einer kosmetischen Zubereitung vorliegt.

Eine weitere Ausführungsform der Erfindung besteht darin, daß der erfindungsgemäße Wirkkomplex in einer kosmetischen Zubereitung zusammen mit einem weiteren Komplex (Komplex II) vorliegt, der gekennzeichnet ist durch eine wäßrige Gelgrundlage und darin enthalten ein verkapselter Extrakt einer wäßrigen Extraktion der Ananas-Frucht und der Rückstand einer wäßrigen Extraktion von Joghurt, wobei die Extraktionen jeweils im Temperaturbereich von 10 bis 30 °C erfolgten und wobei das Verhältnis Ananas-Extrakt zu Joghurtextraktrückstand im Bereich von 20:80 bis 80:20 liegt.

Der Ananas-Extrakt ist vorteilhaft in Liposomen verkapselt, insbesondere in Phospholipid-Liposomen.

Das Verhältnis Ananas-Extrakt zu Joghurtextraktrückstand liegt im Bereich von 40:60 bis 60:40.

Der Komplex II enthält vorteilhaft einen Anteil von Bromelin im Bereich von 0,1 bis 1 Gew-%.

Der Komplex II kann in der kosmetischen Zubereitung in einem Anteil von 0,1 bis 10 Gew-% vorliegen, bezogen auf die Zubereitung.

Zum Nachweis des Synergismus für den erfindungsgemäßen Wirkkomplex ist als in vitro-Test ein Anti-Stress-Test bei UV-Aktivität anwendbar. Dabei wird eine Kultur von dermalen Fibroblasten aus explantierter normaler Humanhaut einem UV-induzierten Stress unterworfen. Die dadurch freigesetzten Mengen des Enzyms Lactico-deshydrogenase (LDH) sind proportional zu den aufgetretenen Zellschädigungen. Dem Kulturmedium zugesetzt wurden z.B. 3 Gew-% Glykogen (Dermosaccharides GY^{®}), und es wurde eine Kontrollreihe ohne diesen Zusatz zum Vergleich angesetzt.

Bei einem für die Kontrollgruppe gesetzten Schutz von 100% ergab der Glykogenzusatz einen Schutz von 128% an überlebenden Zellen 4 Tage nach der UV-Bestrahlung.

In gleicher Weise erfolgte ein Zusatz von 2 Gew-% Kreatin zur Kulturflüssigkeit, und man erhielt gegenüber der Kontrollgruppe einen Schutz von 110%.

Eine Kulturflüssigkeit, die 3 Gew-% Glykogen und 2 Gew-% Kreatin enthielt, zeigte einen Schutz von 153% und lag damit deutlich über dem zu erwartenden summarischen Ergebnis. Die Werte sind jeweils Mittelwerte von 5 Untersuchungen.

Der weitere Zusatz von 1,5 Gew-% β-1,3-Glucan aus dem oben genannten Ultraschallaufschluß von Bäckerhefe erhöhte den Schutz auf 171%.

Ein weiterer Synergismus zeigte sich bei der Oxygraphie, der Stimulierung des Sauerstoffverbrauches bei einem Zellhomogenat von Ephithelzellen. Gegenüber einem als 100% gesetzten Sauerstoffverbrauch für die Rontrollflüssigkeit (Puffer) ergab ein Zusatz von 3 Gew-% Glykogen zur Kontrollflüssigkeit eine Erhöhung des Sauerstoffverbrauches auf 135%. 2 Gew-% Kreatin erhöhten den Sauerstoffverbrauch auf 119%. 3 Gew-% Glykogen und 2 Gew-% Kreatin zusammen erhöhten den Sauerstoffverbrauch auf 174% .

Durch den Zusatz von 1,5 Gew-% β-1,3-Glucan aus dem oben genannten Ultraschallaufschluß von Bäckerhefe wurde der Sauerstoffverbrauch auf 191% erhöht.

Die Ergebnisse zeigen deutlich das Auftreten eines nicht zu erwartenden Synergismus, der zu einer deutlich verbesserten Zellrevitalisierung führt.

Die Erfindung soll nachstehend durch Beispiele näher erläutert werden. Alle Angaben erfolgen in Gewichtsprozent, sofern nichts anderes angegeben ist.

### Beispiel 1 Herstellung des Wirkkomplexes

| **Beispiel 1A** | |
|---|---|
| Creapure Pyruvate^{®} | 9 |
| Phospholipon^{®}80 | 5 |
| Carbomer | 2 |
| Wasser | ad 100 |
| Dermosaccharides.GY^{®} (Glykogen) | 5 |
| Triethanolamin (TEA) | 0,2 |

Kreatinpyruvat und das Phospholipid wurden miteinander vermischt und bei hohen Umdrehungszahlen von etwa 15000 U/min bis zum Erhalt von Liposomen vermischt. Separat wurde das Gel in Wasser eingerührt und ebenfalls homogenisiert. Danach wurde das Homogenisat von Kreatinderivat und Phospholipid in das wäßrige Gel eingerührt und mit TEA neutralisiert. Zu diesem Gemisch wurde Glykogen und gegebenenfalls das aus Hefe gewonnene Glucan unter Rühren gegeben und bei 10.000 U/min bis zur Homogenität homogenisiert.

### Beispiel 1b) bis 1e)

Das Kreatinpyruvat wurde ersetzt durch 8 % Kreatinphosphat (Beispiel 1b), 5 % Kreatincitrat (1c), 3 % Kreatinphosphat (1d), wobei in Beispiel 1d) zusätzlich 1,5 % Glucan aus dem genannten Hefe-Aufschlußverfahren zugesetzt wurde, sowie 7 % reines Kreatin (Beispiel 1e).

### Beispiel 2 Kosmetische Creme

| **Phase A** | |
|---|---|
| Wasser | ad 100 |
| Propylene Glycol | 2 |
| Xanthan Gum | 0,5 |

| **Phase B** | |
|---|---|
| Stearine Acid | 0,5 |
| Stearic Acid | 1 |
| Cetyl Alcohol | 2 |
| Cetearyl Alcohol | 5 |

| **Phase C** | |
|---|---|
| Vitamin E | 1 |
| Wirkkomplex gemäß Beispiel 1a) | 2 |
| Parfüm | 0,5 |
| Konservierungsmittel | 1 |

Die Phasen A und B wurden separat auf etwa 80 °C erwärmt. Unter Rühren wurde die Phase B zur Phase A gegeben, und 20 Minuten wurde das Gemisch homogenisiert und unter Rühren auf etwa 35 °C abgekühlt. Dann erfolgte die Zugabe der Phase C und ein Homogenisieren. Unter Rühren wurde das Gemisch auf 28-30 °C abgekühlt.

### Beispiel 3 Körperlotion

| **Phase A** | |
|---|---|
| Wasser | ad 100 |
| Propylene Glycol | 5 |
| Xanthan Gum | 0,1 |

| **Phase B** | |
|---|---|
| PEG 40 Stearate | 1,5 |
| Cetyl Alcohol | 0,5 |
| Cetearyl Alcohol | 0,5 |

| **Phase C** | |
|---|---|
| Wirkkomplex gemäß Beispiel 1e) | 12 |
| Silicon Oil | 2,5 |
| Parfüm | 0,5 |
| Konservierungsmittel | 1 |

Es wurde wie im Beispiel 2 gearbeitet.

### Beispiel 4 Sensitiv-Creme

| **Phase A** | |
|---|---|
| Wasser | ad 100 |
| Glycerine | 3 |
| Crosspolymer | 0,2 |

| **Phase B** | |
|---|---|
| Steareth 21 | 3,5 |
| Steareth 2 | 2,5 |
| Cetyl Alcohol | 2 |

| **Phase C** | |
|---|---|
| Triethanolamin | 0,2 |

| **Phase D** | |
|---|---|
| Dicapryl Ether | 5 |
| Konservierungsmittel | 1, |

| **Phase E** | |
|---|---|
| Wirkkomplex gemäß Beispiel 1c) | 10 |
| Asymmetrische lamellare Aggregate | |
| gemäß Beispiel 1 von US-A-5643601 | 5 |
| Parfüm | 0,8 |

Die Phasen A und B wurden separat auf etwa 70 °C erwärmt. Unter Rühren wurde die Phase B zur Phase A gegeben, und 20 Minuten wurde das Gemisch homogenisiert und unter Rühren auf etwa 50 °C abgekühlt. Dann erfolgte die Zugabe der Phase C und es wurde auf 40 °C abgekühlt. Anschließend wurde die Phase D hinzugegeben, homogenisiert und das Gemisch bei etwa 35 °C mit der Phase E versetzt und gut verrührt.

### Beispiel 5 Make-up

| **Phase A** | |
|---|---|
| Wasser | ad 100 |
| Glycerine | 2,5 |
| Crosspolymer | 0,3 |

| **Phase B** | |
|---|---|
| Steareth 21 | 2,3 |
| Steareth 2 | 1,8 |
| Cetyl Alcohol | 3,8 |

| **Phase C** | |
|---|---|
| Triethanolamin | 0,3 |

| **Phase D** | |
|---|---|
| Silicone | 5 |
| Konservierungsmittel | 1 |

| **Phase E** | |
|---|---|
| Wirkkomplex gemäß Beispiel 1b) | 19 |
| Hartmagnetische Teilchensuspension gemäß Beispiel 1 von US-A-5800835, Teilchengröße 180 nm | 1 |
| Parfüm | 0, 6 |
| Farbpigmente | 5 |

Es wurde wie im Beispiel 4 gearbeitet.

### Beispiel 6 Dusch-Gel

| | |
|---|---|
| Wasser | ad 100 |
| Crosspolymer | 0,5 |
| Sodium Laureth Sulfate | 20 |
| Cocoamidopropyl Betaine | 10 |
| Quaternium 80 | 1 |
| Wirkkomplex gemäß Beispiel 1d) | 8 |
| Komplex II in Liposomen¹ | 1 |
| Parfümöl | 1 |
| Konservierungsmittel | 1 |

Die Rohstoffe wurde bei Raumtemperatur (etwa 25 °C) nacheinander zu einem Gemisch verrührt.
¹ Ananas-Fruchtextrakt und Rückstand von wäßrigem Joghurt-Extrakt, Verhältnis 20:80; Bromelingehalt 0,4 Gew-% des Komplexes II; hergestellt gemäß Beispiel 3 von DE-A-10042576.

## Patentansprüche

1. Revitalisierender Wirkkomplex für die Haut, **dadurch gekennzeichnet, daß** der Komplex besteht aus
- 0,5 bis 9 Gew-% Kreatin oder einem Kreatinderivat
- 0,1 bis 40 Gew-% wasserlöslichem Glykogen
- 0,1 bis 10 Gew-% eines Phospholipids
- 0,1 bis 5 Gew-% eines kosmetisch annehmbaren Gels, der Rest zu 100 % Wasser,
in homogener Verteilung des Gemisches Kreatin, Glykogen und Phospholipid in dem wäßrigen Gel.

2. Wirkkomplex nach Anspruch 1, **dadurch gekennzeichnet, daß** er zusätzlich 0,05 bis 10 Gew-% eines β-1,3-Glucans enthält, das durch ein schonendes Ultraschall-Aufschlußverfahren von Hefe gewonnen wurde.

3. Kosmetische Zubereitung, enthaltend einen Wirkkomplex nach Anspruch 1 oder 2, worin der Wirkkomplex in einem Anteil von 0.01 bis 22 Gew-% enthalten ist, bezogen auf das Gesamtgewicht der Zubereitung.

4. Zubereitung nach Anspruch 3, worin der Wirkkomplex in einem Anteil von 0,5 bis 12 Gew-% enthalten ist.

5. Zubereitung nach Anspruch 3, worin der Wirkkomplex zusammen mit 0,5 bis 15 Gew-% mit Sauerstoff beladenen asymmetrischen lamellaren Aggregaten aus Fluorcarbonen und Phospholipiden vorliegt.

6. Zubereitung nach Anspruch 3, worin diese 0,5 bis 10 Gew-% hartmagnetische Einkristallen aus Bariumhexaferrit mit einer Koerzitivfeldstärke von 3000 bis 5000 Oerstedt und mit Korngrößen im Bereich von 50 bis 250 nm enthält, und gegebenenfalls 0,5 bis 15 Gew-% mit Sauerstoff beladene asymmetrische lamellare Aggregate aus Fluorcarbonen und Phospholipiden.

7. Zubereitung nach Anspruch 3, worin diese 0,1 bis 10 Gew-% eines weiteren Komplexes enthält, bestehend aus a) einer wäßrigen Gelgrundlage und darin enthalten ein verkapselter Extrakt einer wäßrigen Extraktion der Ananas-Frucht, und b) dem Rückstand einer wäßrigen Extraktion von Joghurt, wobei die Extraktionen jeweils im Temperaturbereich von 10 bis 30 °C erfolgten und wobei das Verhältnis Ananas-Extrakt zu Joghurtextraktrückstand im Bereich von 20:80 bis 80:20 liegt.

8. Zubereitung nach einem der Ansprüche 3 bis 7, worin sie als kosmetische Gele, Cremes, Lotionen, Make-up's oder Stifte vorliegt.

## Claims

1. A revitalising active complex for the skin, which complex comprises
- 0.5 to 9 wt. % of creatine or a creatine derivative
- 0.1 to 40 wt. % of water-soluble glycogen
- 0.1 to 10 wt. % of a phospholipid
- 0.1 to 5 wt. % of a cosmetically acceptable gel, water making up the remainder of the 100 wt. %,
the said mixture of creatine, glycogen and phospholipid being homogeneously distributed in the aqueous gel.

2. An active complex according to claim 1, wherein the said complex contains additionally 0.05 to 10 wt. % of a β-1,3-glucan which has been obtained from yeast by means of a gentle ultrasonic decomposition process.

3. Cosmetic preparation comprising an active complex according to claim 1 or 2 wherein the said complex is contained in cosmetic preparations in an amount ranging from 0.01 to 22 wt. % relative to the total weight of the preparation.

4. Preparation according to claim 3, wherein the said complex is contained in cosmetic preparations in an amount ranging from 0.5 to 12 wt. %.

5. Preparation according to claim 3, wherein the said complex is provided in a cosmetic preparation along with 0.5 to 15 wt. % of asymmetric lamellar aggregates which consist of fluorocarbons and phospholipids and which are loaded with oxygen.

6. Preparation according to claim 3, wherein the said preparation comprises 0.5 to 10 wt. % of magnetically hard single-crystals consisting of barium hexaferrite and having a coercivity of between 3,000 and 5,000 oerstedts and particle sizes ranging from 50 to 250 nm and, optionally, 0.5 to 15 wt. % of asymmetric lamellar aggregates which consist of fluorocarbons and phospholipids and are loaded with oxygen.

7. Preparation according to claim 3, comprising 0.1 to 10 wt. % of another complex consisting of a) an aqueous gel base which contains an encapsulated extract obtained by means of an aqueous extraction of pineapple fruit and b) the residue of an aqueous extraction of yoghurt, both extraction processes being carried out at a temperature ranging from 10 to 30 °C and the ratio of pineapple extract to yoghurt residue ranging from 20:80 to 80:20.

8. Preparation according to one of claims 3 through 7, wherein it is a cosmetic gel, cream, lotion, make-up or stick.

## Revendications

1. Complexe actif revitalisant pour la peau, **caractérisé en ce que** le complexe comprend
- 0,5 à 9 % en poids de créatine ou d'un dérivé de la créatine
- 0,1 à 40 % en poids de glycogène hydrosoluble
- 0,1 à 10 % en poids d'un phospholipide
- 0,1 à 5 % en poids d'un gel pour cosmétiques
complétés à 100 % avec de l'eau, avec une dispersion homogène du mélange créatine, glycogène et phospholipide dans le gel aqueux.

2. Complexe actif selon la revendication 1, **caractérisé en ce qu'**il contient en plus 0,05 à 10 % en poids d'un β-1,3-glucane obtenu par un procédé doux de dissolution de levure par des ultrasons.

3. Préparation cosmétique contenant un complexe actif selon la revendication 1 ou 2, dans laquelle le complexe actif se trouve dans une proportion de 0,01 à 22 % en poids par rapport au poids total de la préparation.

4. Préparation selon la revendication 3, dans laquelle le complexe actif se trouve dans une proportion de 0,5 à 12 % en poids.

5. Préparation selon la revendication 3, dans laquelle le complexe actif est présent avec 0,5 à 15 % en poids d'agrégats lamellaires asymétriques chargés en acide, à base de fluorocarbones et de phospholipides.

6. Préparation selon la revendication 3, contenant 0,5 à 10 % en poids de monocristaux magnétiques durs en hexaferrite de baryum avec une intensité de champs coercitif de 3 000 à 5 000 Oersted et avec une granulométrie dans la plage de 50 à 250 nm, et le cas échéant 0,5 à 15 % en poids d'agrégats lamellaires asymétriques chargés en acide, à base de fluorocarbones et de phospholipides.

7. Préparation selon la revendication 3, contenant 0,1 à 10 % en poids d'un autre complexe composé a) d'une base de gel aqueux renfermant un extrait encapsulé d'une extraction aqueuse de l'ananas-fruit, et b) du résidu d'une extraction aqueuse de yaourt, sachant que les extractions ont chacune eu lieu dans une plage de température de 10 à 30°C et que le rapport extrait d'ananas sur résidu d'extrait de yaourt se situe dans la plage 20:80 à 80:20.

8. Préparation selon l'une quelconque des revendications 3 à 7, se présentant comme gels, crèmes, lotions, maquillages ou crayons cosmétiques.
